# NEW EUROPEAN PATENT SPECIFICATION

(11) **EP 1 767 185 B2**
(45) Date of publication and mention of the opposition decision: **28.11.2018**
(45) Mention of the grant of the patent: 27.10.2010
(21) Application number: 05291976.8
(22) Date of filing: 23.09.2005
(51) Int. Cl.: A61K 8/11, A61K 8/81, A61Q 5/12, D06M 13/00, A61Q 5/02, A61Q 5/06, A61Q 13/00, C11D 3/50

(54) **Core shell capsules containing an oil or waxy solid**
Kern/Schale-Kapseln enthaltend ein Öl oder einen wachsartigen Feststoff
Capsules du coeur-coquille comprenant une huile ou un matière crieux

(43) Date of publication of application: 28.03.2007
(73) Proprietor: Takasago International Corporation, Tokyo-to 144-8721 (JP)
(72) Inventor: Warr, Jonathan, 75017 Paris (FR); Fraser, Stuart, Little Neston Cheshire CH64 4DH (GB); Aussant, Emmanuel, 75011 Paris (FR); Masakatsu, Unno, 92210 Saint Cloud (FR)
(74) Representative: Nevant, Marc

(56) References cited:
- EP-A- 0 328 937
- EP-A- 0 965 326
- EP-A- 1 533 415
- EP-A2- 1 533 364
- WO-A-02/074430
- WO-A-2004/016234
- WO-A-2005/017085
- WO-A1-98/28396
- GB-A- 2 073 132
- US-A- 5 500 138
- US-A- 5 500 223
- US-A1- 2004 115 091
- <<<N O N - C I T E D D O C U M E N T>>> "3. Auflage" In: Janistyn H.: "Taschenbuch der modernen Parfümerie und Kosmetik", 1966, Wissenschaftliche Verlagsgesellschaft M.B.H, Stuttgart page 162ff,

## Description

### The Technical Field of the Invention

The invention relates to compositions to be incorporated into the core of core shell type capsules to control the delivery and release of fragrance and optionally other benefit agents when used as components within household, personal care and cosmetic products. Chiefly, the invention relates to core shell capsules made by the condensation of formaldehyde with melamine and/or urea as the major monomers around an emulsion of the core materials.

The invention further relates to the use of these capsules in household, personal care, and cosmetic products.

### Background of the Invention

Delivery of a lingering or substantive fragrance is a common benefit of many household, personal care and cosmetic products. Delivery of benefit agents to surfaces by direct inclusion into products is often very inefficient and researchers have searched for improved delivery methods. For fragrance the selection of specific fragrance components can improve the delivery of perfume as illustrated in US patent 5,500,138 which claims a percentage of high ClogP materials in a fragrance for fabric conditioners. Encapsulating a benefit agent and/or fragrance in order to control its delivery and release behaviour is another technology which has been explored as in US patent 4,209,417 in which fragrance is emulsified within a starch polymer to be protected in storage and released during the laundry process or US patent 4,152,272 which teaches the incorporation of perfume into wax capsules applied from a fabric conditioner thereby protecting the fragrance in storage and during laundering leading to greater deposition on the dry fabric. US patent 4,145,184 describes the incorporation of fragrance into a core shell capsule which is entirely surrounded by a polymer, thus the perfume is protected throughout the laundry process and released when the capsule is ruptured. International patent application WO99/65458 teaches the formulation of capsule ingredients based on selection of materials having preferred volatilities (boiling point), hydrophobicity (ClogP) and human olfactory sensitivity (perception threshold). Notwithstanding all these efforts there is still a need to improve the delivery of benefit agents particularly from laundry products and especially from detergent powders.

A selection of fragrance ingredients has now been found which are a) capable of being incorporated within specific capsules, b) chemically stable on storage within these capsules and c) have suitable physical properties to be appreciated to their greatest extent when the capsules are ruptured and the contents released.

### Summary of Invention

The present invention relates to a core shell capsule as defined in the claims and containing in the core an oil or waxy solid, said oil or solid waxy comprising by weight:
- 80-100% of a perfume composition , which is a mixture of at least two perfume ingredients, wherein:
   - aldehydes, including alpha beta unsaturated aldehydes, constitute 0-20% preferably 0-10% more preferably 0-5% and even more preferably 0-1% by weight of the perfume composition;
   - primary or secondary amines constitute 0-10% preferably 0-1% by weight of the perfume composition;
   - 0-25%, preferably 0-20% by weight of the perfume composition has a ClogP >4.0;
   - 0-20%, preferably 0-15% by weight of the perfume composition has a ClogP >5.0;
   - 0-20% and preferably 0-10% by weight of the perfume composition has a ClogP <2.0;
- 0-20% by weight of benefit agents other than perfume ingredients.

The benefit agents other than perfume ingredients, which should also satisfy above conditions a) and b), are preferably selected among the group consisting of malodour counteracting agents, essential oils, aromatherapeutic materials, chemaesthetic agents vitamins, insect repellents, UV absorbers, antioxidants and agents which improve the capsule properties such as:
a) by stabilising the emulsion during capsule manufacture,
b) by reducing leakage from the capsule,
c) by improving capsule hardness.

### Detailed Description of the Invention

### Perfume Composition

In the context of this specification a "perfume composition" , which is also named "fragrance" as defined below is an essential part of the invention. The term "perfume composition" means any odoriferous material or any material which acts as a malodor counteractant. A wide variety of chemicals are known for perfumery uses, including materials such as alcohols, ketones, esters, ethers, nitriles, and the like. Without wishing to be limited, normally in most cases, the perfume compounds will have molecular weights of less than 400 mass units to ensure sufficient volatility and will not contain strongly ionizing functional groups such as sulphonates, sulphates, or quaternary ammmonium ions.

Naturally occurring plant and animal oils and exudates or oils and exudates identical to those found in the nature, comprising complex mixtures of various chemical components are also known for use as perfumes, and such materials can be used herein. Perfume compositions of the present invention can be relatively simple in their composition with a minimum of two perfume or fragrance ingredients or can comprise highly complex mixtures of natural and synthetic chemical components, chosen to provide any desired odour. Perfume ingredients are described more fully in S. Arctander, Perfume Flavors and Chemicals. Vols. I and II, Aurthor, Montclair, N.J., and the Merck Index, 8th Edition, Merck & Co., Inc. Rahway, N.J., both being incorporated herein by reference.

According to one aspect of the invention it has been found that aldehydes not only react to some extent during the preparation of the capsules but surprisingly they continue reacting over time on storage within the capsule itself to an extent which may make the fragrance olfactively unacceptable. Despite the general view that aldehydes are reactive species some aldehydes e.g. lilial, cyclamen aldehyde and hexyl cinnamic aldehyde are frequently used at quite high levels in fragrances for laundry products and are stable in these formulations. The perfume composition of the present invention must restrict the level of total aldehydes including alpha, beta unsaturated aldehydes to less than 20% by weight, preferably less than 10% and even more preferably less than 1% of the perfume composition.

It has also been found that although an excess of water soluble amines is added at the end of the capsule manufacture to remove formaldehyde, the amines present as core components show a surprising degree of stability with the capsule. Thus, the perfume composition of the invention must contain less than 10% by weight, and more preferably less than 1% of primary and secondary amines.

A further aspect of the invention is that the capsule should contain more than 80% of perfumery ingredients. Whilst economically it would seem obvious to incorporate as much active ingredients as possible into each capsule, for many practical reasons, associated with emulsion stability, capsule integrity etc., many capsules contain other ingredients e.g. solvents, hardeners which substantially dilute the fragrance and benefit agents.

Related to the above is the realization that the fragrance no longer plays a role in deposition so the need to choose a proportion of high ClogP (Calculated logP) materials as taught in US patents 5,652,206 and 5,500,138 for improved delivery and fragrance longevity is no longer required. Indeed, it is preferable if more volatile ingredients are selected for the fragrance to give maximum perfume impact. Thus fragrance compositions of the invention require less than 25% by weight of perfume ingredients preferably less than 20% with ClogP>4 and less than 20% with ClogP <2.

ClogP refers to the octanol/water partitioning coefficient (P) of fragrance ingredients. The octanol/water partitioning coefficient of a perfume ingredient is the ratio between its equilibrium concentrations in octanol and in water. The partitioning coefficients of perfume ingredients are more conveniently given in the form of their logarithm to the base 10, logP. Thus the perfume ingredients of this invention have logP of about 1.5 and higher preferably in the range 2.5 to 5. The logP of many perfume ingredients has been reported; for example, the Pomona92 database, available from Daylight Chemical Information Systems, Inc. (Daylight CIS), Irvine, Calif., contains many, along with citations to the original literature. However, the ClogP values reported herein are most conveniently calculated by the "CLOGP" program available within the Chemoffice Ultra Software version 9 available from CambridgeSoft Corporation, 100 CambridgePark Drive, Cambridge, MA 02140 USA or CambridgeSoft Corporation, 8 Signet Court, Swanns Road, Cambridge CB5 8LA UK. The ClogP values are preferably used instead of the experimental logP values in the selection of perfume ingredients which are useful in the present invention. For natural oils or extracts the composition of such oils can be determined by analysis or using the compositions published in the ESO 2000 database published by BACIS (Boelens Aroma Chemical Information Service, Groen van Prinsterlaan 21, 1272 GB Huizen, The Netherlands).

Preferably, the oil or waxy solid contains 0-1 % of perfume ingredients, which are selected among :
i. the aldehydes selected among the group consisting of of amyl cinnamic aldehyde; citral (CAS 005392-40-5); hydroxy-citronellal; cinnamic aldehyde; hydroxymethylpentyl-cyclohexenecarboxaldehyde; 2-(4-tert-butylbenzyl) propionaldehyde; hexyl cinnamic aldehyde; phenyl acetaldehyde; trans-2-heptenal; 2,4-dihydroxy-3-methyl benzaldehyde; Benzaldehyde; Crotonaldehyde E (CAS 123-73-9); and furfural (CAS 99-01-1);
ii. the perfume ingredients having a ClogP > 4 selected among the group consisting of of Benzyl salicylate, Benzyl cinnamate, Farnesol (CAS 4602-84-0), d-Limonene. I-Limonene, D, L-Limonene (racemic), 3-Methyl-4-(2,6,6-trimethyl-2-cyclohexen-1-yl)-3-buten-2-one, Cyclowood (CAS 13019-04-0), Polysantol (CAS 107898-54-4),
iii. the perfume ingredients having a ClogP < 2 selected among the group consisting of by benzyl alcohol, Cinnamyl alcohol, Coumarin; Anisyl alcohol; Acetal E71 (CAS 105-57-7), acetophenone; Sec-Butyl acetate; tert-Butyl acetate; n-Butyl acetate; iso-Butyl acetate, p-Cresol; Ethyl acetate; Ethyl propionate; Propyl acetate; Ethyl propionate; Propyl acetate; Benzyl cyanide.

Since it is inherent in the success of this invention that more fragrance will be deposited on surfaces and that the local concentration around ruptured capsules will be quite high, the composition of the capsule core must take into account the less desirable characteristics of some fragrance materials such as persistence in the environment, accumulation in aquatic organisms, and toxic, allergenic or irritant effects with some humans.

In general, since the capsules will deliver fragrance more efficiently to the surface fewer capsules and hence less fragrance is needed to achieve a desired fragrance effect, so the overall environmental load is reduced. However the greater concentration on skin or in close proximity to the skin requires additional care to formulate the core composition using only ingredients known to be safe in such a context. Among the materials known to have undesirable characteristics and therefore preferably excluded from the invention perfume compositions are nitro musks as exemplified by musk ambrette CAS 83-66-9, and musk ketone CAS 81-14-1, polycyclic musks typified by Galaxolide CAS 1222-05-5 and Tonalid CAS1506-02-1, cashmeran, geranyl nitrile, safrole, estragol, methyl eugenol, halogen containing perfumery materials. Solvents especially the phthalate esters and carbitol ethers defined as R-(OCH₂CH₂)ₙ-OR¹ where n= 1,2 or 3 R= (C₁-C₇) alkyl or phenyl or alkyl substituted phenyl and R¹ is H or (C₁-C₇)alkyl.

Materials listed in Annex 1 of the Dangerous Substances Directive (67/548/EEC) or any of its amendments or ATPs (Adaptation to Technical Progress), or classified as R43 in their safety data sheet are optionally restricted to less than 1% of the core composition, preferably less than 0.1% by weight, more preferably below 0.001%, and even more preferably below the analytical detection limit.

In addition any materials classified as very toxic or toxic are preferably excluded from the core composition. Those fragrance ingredients alleged to be allergenic substances within the 7th amendment of the Cosmetic Directive, Directive 2003/15/EC (7th amendment to Directive 76/768/EEC) and the Detergent Regulations (2004/648/EEC) are optionally restricted to below 1% by weight, preferably below 100ppm and more preferably below 10ppm of the core composition. These Directives are also amended via ATPs, for example the 26^{th} Commission Directive 2002/34/EC. The core composition is preferably formulated so as not to require any form of classification or warning phrase, especially classification Xi or Xn due to for example the presence of amounts of raw materials classified as R43 "sensitisasion by skin contact", R36 "irritating to eyes", R38 "irritating to skin" or R21 "Harmful in contact with skin" under the Dangerous Preparations Directive (99/45/EEC).

It is sometimes found that oxidation of certain raw materials can lead to the formation of peroxides, and that these peroxides have some health concerns. The SCCNFP (Scientific Committee on Cosmetic Products and Non Food Products for Consumers) in their opinion SCCNFP/0392/00,final, give a number of raw materials where there is concern. The oil or waxy solid has preferably a peroxide value of 0-20 millimoles peroxide / litre, preferably 0-10 millimoles peroxide / litre, and even more preferably 0-1 millimoles peroxide / litre. In particular it is desirable that if limonene (d-, l-, and dl-), and natural products containing substantial amounts of limonene, are used, they should have a peroxide value of less than 20 millimole peroxide per litre. The methods for measuring peroxide value are well known to those skilled in the art, and a method is published by the FMA (Fragrance Material Association).

Typical perfume compositions herein can comprise, for example, woody/earthy notes containing as perfume ingredients synthetic materials and natural extracts such as sandalwood oil, or patchouli oil and the like. The perfumes herein can be of a light, floral fragrance, e.g., rose, violet, jasmine, lily and the like. The perfume compositions herein can be formulated to provide desirable fruity odors, e.g., lime, lemon, orange, berry fruits or peach and the like.

In short, any chemically compatible material which exudes a pleasant or otherwise desirable odor can be used in the perfumed capsules herein to provide a desirable odor when applied to fabrics.

Table 1 below lists some perfume ingredients which have ClogP values, calculated using Chemoffice Ultra Version 9, between 2.0 and 5.0 and which comply with the requirements of the present invention. The values were found to be essentially identical to those obtained using Daylight CLogP (version 4.9).

**Table 1**

| Name | ClogP | CAS n° |
|---|---|---|
| Laevo carvone | 2.01 | 6485-40-1 |
| Geraniol | 2.97 | 106-24-1 |
| Cis Jasmone | 2.64 | 588-10-8 |
| Alpha Terpineol | 2.63 | 98-55-5 |
| Eugenol | 2.34 | 97-53-0 |
| Methyl cinnamate | 2.46 | 103-26-4 |
| Methyl dihydrojasmonate | 2.91 | 24851-98-7 |
| Beta methyl naphthyl ketone | 2.76 | 93-08-3 |
| Iso bomyl acetate | 4.04 | 125-12-2 |
| Carvacrol | 3.35 | 499-75-2 |
| Para cymene | 4.07 | 99-87-6 |
| Dihydromyrcenol | 3.04 | 18479-58-8 |
| Geranyl acetate | 3.91 | 105-87-3 |
| Unalyl acetate | 3.70 | 115-95-7 |
| Vertenex | 4.06 | 32210-23-4 |

Table 2 below lists examples of materials, widely used in fragrances for household, personal care and cosmetic products, the levels of which are restricted within the invention.

**Table 2**

| Name | ClogP | CAS n° |
|---|---|---|
| Hydroxycitronellal | 1.54 | 107-75-5 |
| Linalool | 2.75 | 78-70-6 |
| Phenyl ethyl alcohol | 1.33 | 60-12-8 |
| Coumarin | 1.41 | 91-64-5 |
| Vanillin | 1.28 | 121-33-5 |
| Citronellol | 3.25 | 106-22-9 |
| d-Limonene | 4.35 | 5989-27-5 |
| Isobutyl quinoline | 3.98 | 93-19-6 |
| Hexyl cinnamic aldehyde | 5.00 | 101-86-0 |
| Lilial | 4.10 | 80-54-6 |
| Galaxolide | 5.74 | 1222-05-5 |
| Cyclamen aldehyde | 3.83 | 103-95-7 |

In both tables the lists are not intended to be exhaustive but are included merely to clarify the definitions.

The invention also encompasses the use of odiferous materials which also act as malodor counteractants. These materials, although termed "perfume ingredients" hereinafter, may have a weak odor but can conceal or reduce any unpleasant odors. Examples of suitable malodor counteractants are disclosed in US patent. 3,102,101 and in US patent 5,554,588.

### Solvents

Olfactively weak or neutral solvents may constitute up to 30% of the capsule core material by weight, preferably less than 20% by weight and more preferably less than 10% by weight. If present they will most likely have been introduced with one or more perfume ingredients. In the perfume industry it is quite common to dissolve solid fragrance materials in a suitable solvent or to dilute powerful materials, used at low levels, with a solvent to facilitate manufacture. Typical solvents include high ClogP materials such as benzyl benzoate, isopropyl myristate, dialkyl adipates, citrate esters such as acetyl triethyl citrate or acetyl tributyl citrate or triethyl citrate or diethyl phthalate or low ClogP materials such as propylene glycol or dipropylene glycol. While these materials could affect fragrance release or emulsion properties during capsule manufacture, at the levels described such effects will be minimal. For the purpose of this patent, when solvent is present, it is considered as an "other benefit agent".

### Other benefit agents

In the context of this specification, "other benefit agen"t means any material capable of being encapsulated in the way described later and which can survive storage to deliver a benefit when used in household, personal care or cosmetic products provided it contains little or no aldehydes, in particular alpha, beta unsaturated aldehydes or primary or secondary amines ; as described previously, i.e. they should satisfy the requirements concerning aldehydes and amines given above for the perfume composition. Benefit agents do not have to conform to the ClogP requirements as outlined for the fragrance ingredients since it is not a necessary feature of the benefit agents that they vapourise to be effective.

Benefit agents include natural extracts or materials which have therapeutic effects as relaxants or stimulants, e.g. aromatherapy oils, whether odiferous or not. Natural oils or plant extracts which are beneficial to skin such as jojoba oil or almond oil are also benefit agents. Vitamins or vitamin derivatives such as ascorbyl palmitate (CAS 137-66-6) tocopheryl acetate (CAS 58-95-7) or retinyl palmitate (CAS 79-81-2) are also benefit agents within this definition. Materials which suppress or reduce malodour and its perception by any of the many mechanisms proposed are benefit agents such as zinc ricinoleate (CAS 13040-19-2). Materials which when added to the emulsion improve the properties of the core emulsion before encapsulation, or the properties of the capsules themselves. Materials which provide a warming or cooling effect such as described in Cosmetics and Toiletries Vol. 120 No 5 p105 by M Erman are also benefit agents. Examples of such agents include but are not limited to: cyclohexane carboxamide N-ethyl-5-methyl-2-(1-methylethyl) known as WS3™ (CAS N" 39711-79-0); N 2,3-trimethyl-2-isopropylbutamide known as WS23™ (CAS 51115-67-4); menthyl lactate (CAS N° 59259-38-0); (-)-menthoxypropane 1,2-diol known as cooling agent 10™ and isopulegol. Materials which act as insect repellents exemplified by ethylbutylacetylaminopropionate known as Merck's IR3535™ (CAS N° 52304-36-6); or N,N-diethyl touamide (CAS N° 134-62-3); or 1-piperidinecarboxylic acid; 2-(2-hydroxyethyl)-1-methylpropyl ester known as Bayrepel™ (CAS N° 119515-38-7); or p-menthane-3,8-diol (CAS N° 42822-86-6) or natural plant oils such as Tea Tree oil, neem oil, citronella oil, or eucalyptus oil are benefit agents. Materials which act as antimicrobial agents as exemplified by triclosan™ (CAS N° 3380-34-5), the methyl-ethyl, propyl and butyl para hydroxy benzoate esters (CAS N° 4247-02-3, 94-26-8,94-13-3, 120-47-8, 99-76-3), 2-phenoxyethanol, 3-iodopropynyl-2-butylcarbamate (CAS N° 55406-53-6), 2-bromo-2-nitropropane-1,3 diol (CAS N° 52-51-7) and natural oils such as clove oil, pine oil, cinnamon oil, and tea tree oil are benefit agents. Materials which act as antioxidants such as butylated hydroxyl toluene or butylated hydroxyanisole or pentaerythrityl tetra- di- t-butyl hydroxyhydrocinnamate, octadecyl di t-butyl-4-hydroxyhydrocinnamate (CAS N° 2082-79-3), tetrabutyl ethylidenebisphenol (CAS N° 35958-30-6) are benefit agents. Materials which act as UV absorbers such as octyl methoxycinnamate, Benzophenone 3, butylmethoxydibenzoylmethane, or benzotriazolyl dodecyl p cresol (CAS N° 6683-19-8), bis ethylhexyloxyphenolmethoxyphenyltriazine are benefit agents. The materials listed above are intended to exemplify the benefit agents but are not intented to limit the benefit agents to this list. Mixtures of the above may also be considered as benefit agents of the invention. Thus it may be advantageous to combine UV absorbers with antioxidants to protect the fragrance ingredients or to combine an anti-fungal agent with a bacteriocide for broader antimicrobial protection. Moreover it is recognized that some materials may exhibit more than one benefit. Thus vitamin E acetate can function as an antioxidant as well as a vitamin precursor.

### Capsule Preparation

Various patents describe compositions and processes for manufacturing aminoplast capsules in the form of a dispersion such as EP 1,246,693 A1, US patent 6,261,483 and US 6,719,931 which are incorporated herein by reference. Without wishing to limit the patent in any way a typical process for preparing a capsule dispersion would include the following steps.

The preparation of an emulsion of the perfume ingredients and any benefit agents or modifiers which may include emulsifying agents or emulsion stabilizers takes place under vigorous agitation.

The first step is the mixing of the above emulsion with melamine-formaldehyde resin (with a melamine:formaldehyde:methanol mixture in the approximate molar ratios 1:3:2 to 1:6:4) and an emulsifier. These monomers may be precondensed or the monomers may be used directly. Some of the melamine can be replaced by urea. In these polymers, the formaldehyde may be partially etherified preferably as the methyl ethers.

Preferably, the shell is constituted of 50-100% formaldehyde-melamine or formaldehyde-melamine-urea or formaldehyde-urea condensation polymers or partially corresponding etherified formaldehyde condensation polymers, preferably as the methyl ethers.

Then, acid is added to the above mixture to adjust to a pH of 3.5 to 6.5 and the temperature raised to 30-45°C. Stirring is allowed to proceed until the dispersion is oil free. Any acid which has no adverse properties may be used in this process, such as for example formic acid or acetic acid.

It is particularly advantageous if the capsules are cured by heating to a temperature between 60°C to 100°C for several hours under moderate stirring.

It is particularly advantageous if during the early phase of curing a further addition of urea, melamine or other amines, or mixtures thereof can be made to reduce the formaldehyde concentration in the finished dispersion, and increase the wall thickness. Typically 10-30% additional melamine and/or urea can be added at this stage, and a particularly advantageous ratio is 5:1 to 1:1 melamine:urea.

Once curing is complete, the temperatrure is reduced to around 50°C, and the dispersion is neutralized before being adjusted to a pH around 9.5.

The final capsule dispersion as shipped should contain less than 0.1% by weight of free formaldehyde or free acetaldehyde measured by GLC or HPLC (standard methods are published by the US Environmental Protection Agency; HPLC requires derivitisation of the formaldehyde), preferably less than 100 ppm (wt/wt) and more preferably less than 10 ppm wt/wt.

It may also be advantageous to incorporate physically or chemically further materials to improve capsule deposition to substrates or to improve deposition selectivity during application or to improve the stability of the dispersion over time during storage. Such materials as cationic polymers or copolymers e.g. polyvinyl imidazole, polysaccharides based on beta 1, 4 linkages such a guar gum, and polyester copolymers such as those sold commercially as soil release polymers for detergents are examples of suitable materials to improve deposition.

Capsules of the above process will generally have a particle size within the range from 1-100 µm, preferably 5-70 µm, depending on the emulsifying conditions. The capsule wall will have a thickness of 0.025-1.0 µm. These parameters are important in the proper functioning of the capsules. If the capsule wall is too thin, the capsules will be too friable for subsequent shipping and handling, if too thick they might not break when required. If capsules are very small the wall material may become an uneconomically large proportion of the capsule. Very large capsules either require thicker walls or the addition of hardeners to the core to prevent breakage in handling both of which reduces the amount of beneficial agent delivered.

The final dispersion may typically contain, by weight, 2.5%-80%, preferably 5%-70% and more preferably 30%-70% capsules dispersed in water. In some forms of the process excess water can be removed to form either a concentrated wet cake or a dry free flowing powder as best suits the subsequent application.

The capsules of the invention may be used in any personal care composition, liquid or solid household cleaning or care compositions or powder detergent composition produced by spray drying capsules in a mixture with inorganic salts, and optionally a binder and/or surfactant.

The capsules of the invention are particularly useful for delivering perfume to surfaces by the method which comprises contacting the surfaces with the above-defined household cleaning or care compositions or detergents.

The present invention will be now disclosed in more details by the following illustrative, but not limitating examples. The perfume compositions used in the examples (perfume compositions n°1 to 5) were prepared by mixing equal parts of each ingredient indicated in Tables 3-7.

### Example 1: preparation of capsules according to the invention

A 2 I cylindrical stirring vessel was fitted with an infinitively adjustable disperser having a standard commercial dispersion disk with a diameter of 50 mm.

It was charged in succession with:
- 400 g of Fragrance (Perfume Composition No 3 below),
- 69 g of a 70% solution of a methylated melamine-formaldehyde resin (molar ratio melamine: formaldehyde : methanol 1:3.9:2.4) with a Brookfield viscosity of 275 mPas and a pH of 8.5,
- 64 g of a 20% solution of poly-2-acrylamido-2-methylpropanesulfonic acid sodium salt as emulsifier (K value 123, Brookfield viscosity 770 mpas),
- 350 g of water,
- 15 g of 10% strength formic acid.

This charge was processed to a capsule dispersion by adjusting the stirring speed to a peripheral speed of approximately 20 ms⁻¹. The temperature was held at about 35°C.

After 60 minutes, the dispersion was oil-free; a particle size of about 5 µm had been established. The stirring speed of the dispersion disk was then reduced to a level sufficient for uniform circulation of the vessel contents.

A cure temperature of 90°C was set, and once reached by injection of hot steam, a feed of a 27% suspension of melamine-urea (ratio 2.5:1, melamine:urea) in formic acid (to adjust pH to pH 4.5) was added to the dispersion of the preformed microcapsules with a constant mass flow rate and was metered in over the course of an hour. A total of 67 g of the suspension of melamine-urea was metered in.

A cure phase of 120 min ensues at 90°C.

After the dispersion had been cooled to about 55°C, it was neutralized with diethanolamine and adjusted to a pH of 9.5 using ammonia.

This gave a uniform capsule dispersion with a solids content of 50% and a viscosity of 83 mPas.

Following the same procedure, capsules were made with the perfume ingredients and optionally the other benefit agents mentioned in Tables 4 to 7.

### Example 2: stability of perfumes ingredients during the encapsulation and after storage for 8 weeks

The stability of perfumery ingredients during capsule preparation as defined in example 1 and after storage for 8 weeks at ambient temperature of the capsules was measured. The test is a measure of the total amount of fragrance in the dispersion, and does not distinguish "encapsulated" fragrance from free fragrance in the supernatant liquid. The results obtained are given in Tables 3 to 7.

The results given in tables 4 and 6 below show that fragrance materials containing an aldehyde function suffer losses during the encapsulation process, and potentially on storage.

The results given in table 7 below show that fragrance raw materials containing an amine function suffer losses during the encapsulation process.

**Table 3**

| Perfume composition n°1 | CAS N° | ClogP | Stability fresh | Stability 2 months |
|---|---|---|---|---|
| Iso amyl alcohol | 123-51-3 | 1.22 | OK | OK |
| Butyl acetate | 123-86-4 | 1.77 | OK | OK |
| Phenyl ethyl alcohol | 60-12-8 | 1.33 | OK | OK |
| Veltol Plus | 4940-11-8 | 1.13 | OK | OK |
| Cinnamic Alcohol | 104-54-1 | 1.61 | OK | OK |
| Beta Caryophyllene | 87-44-5 | 6.45 | OK | OK |
| Raspberry Ketone | 5471-51-2 | 1.22 | OK | OK |
| Exaltolide | 106-02-5 | 5.34 | OK | OK |
| Hexadecanolide | 109-29-5 | 5.91 | OK | OK |

**Table 4**

| Perfume composition n°2 | CAS No | ClogP | Stability fresh | Stability 2 months |
|---|---|---|---|---|
| Linalool | 78-70-6 | 2.75 | OK | OK |
| Citronellal | 106-23-0 | 3.26 | 30% loss | 50% loss |
| Benzyl Acetate | 140-11-4 | 1.96 | OK | OK |
| C-10 Aldehyde | 112-31-2 | 4.01 | 80% loss | 80% loss |
| Citral | 5392-40-5 | 2.95 | No loss | 100% loss |
| Cinnamic Aldehyde | 104-55-2 | 2.05 | 20% loss | 80% loss |
| Verdox | 88-41-5 | 4.06 | OK | OK |
| Damascenone | 23696-85-7 | 4.27 | OK | OK |
| Cyclacet | 5413-60-5 | 2.88 | OK | OK |
| Beta Ionone | 14901-07-6 | 3.71 | OK | OK |
| Iso Super E | 54464-57-2 | 4.85 | OK | OK |
| Musk T | 105-95-3 | 3.02 | OK | OK |
| Cooling Agent 10 (other benefit agent) | 87061-04-9 | 2.42 | OK | OK |

The composition of Table 4 illustrates an oil according to the invention containing a perfume composition and an other benefit agent.

**Table 5**

| Perfume composition n°3 | CAS No | ClogP | Stability fresh | Stability 2 months |
|---|---|---|---|---|
| Alpha Pinene | 80-56-8 | 4.70 | OK | OK |
| Eucalyptol | 470-82-6 | 2.83 | OK | OK |
| Dihydromyrcenol | 18479-58-8 | 3.03 | OK | OK |
| Linalool | 78-70-6 | 2.75 | OK | OK |
| Benzyl acetate | 140-11-4 | 1.96 | OK | OK |
| Ethyl benzoate | 93-89-0 | 2.64 | OK | OK |
| C-10 Alcohol | 112-30-1 | 4.00 | OK | OK |
| Dimethylbenzyl carbinyl acetate | 151-05-3 | 2.99 | OK | OK |
| Phenylethyl-2-methylbutyrate | 24817-51-4 | 3.74 | OK | OK |
| Hexyl Benzoate | 6789-88-4 | 4.76 | OK | OK |
| Acetyl iso eugenol | 93-29-8 | 2.48 | OK | OK |

**Table 6**

| Perfume composition n°4 | CAS No | ClogP | Stability fresh | Stability 2 months |
|---|---|---|---|---|
| Iso amyl alcohol | 123-51-3 | 1.22 | OK | OK |
| Butyl acetate | 123-86-4 | 1.77 | OK | OK |
| Cis 3-hexenol | 928-96-1 | 1.40 | OK | OK |
| Benzaldehyde | 100-52-7 | 1.49 | OK | OK |
| Phenyl ethyl alcohol | 60-12-8 | 1.33 | OK | OK |
| Veltol plus | 4940-11-8 | 1.13 | OK | Slight loss |
| Cinnamic Alcohol | 104-54-1 | 1.61 | OK | OK |
| Vanillin | 121-33-5 | 1.28 | OK | Slight loss |
| Coumarin | 91-64-5 | 1.41 | OK | OK |

**Table 7**

| Perfume composition n°5 | CAS No | ClogP | Stability fresh | Stability 2 months |
|---|---|---|---|---|
| Acetophenone | 98-86-2 | 1.58 | OK | OK |
| Methyl salicylate | 119-36-8 | 2.33 | OK | OK |
| Veltol Plus | 4940-11-8 | 1.13 | 30% loss | 50% loss |
| Koavona | 81786-73-4 | 3.48 | OK | OK |
| Phenyl acetaldehyde dimethyl acetal | 101-48-4 | 1.57 | OK | OK |
| Methyl anthranilate | 134-20-3 | 2.12 | 30% loss | 30% loss |
| Eugenol | 97-53-0 | 2.40 | OK | OK |
| Hedione | 24851-98-7 | 2.91 | OK | OK |
| Orbitone | 54464-57-2 | 5.24 | OK | OK |
| Ambretone | 37609-25-9 | 5.97 | OK | OK |

### Example 3

The encapsulated dispersion as defined in example 1 was further analysed to distinguish which perfume raw materials were well encapsulated and which materials were largely present in the aqueous, supernatant, phase. This analysis showed that the raw materials with ClogP <2 were predominantly present in the aqueous phase, and were not well encapsulated.

### Example 4: Solid Household Cleaning Compositions

The capsule dispersions described above can be added directly to certain powders, and in particular to certain laundry detergent powders. A key criterion as to whether it is possible to add the dispersion or not is whether the powder properties are not grossly affected by the water which is associated with the dispersion (typically 50% by weight). To determine the suitability of a powder a simple "agglomeration test" has been defined which simply uses water, representing the water which would be present in a capsule dispersion.

This "agglomeration test" consists of taking 100g of powder, which is preferably free from bleaching agents, and adding it to the mixing bowl of a Kenwood Chef mixer fitted with a balloon whisk mixing attachment. The powder is constantly agitated at a power setting sufficient to mix the powder (approximately 70% of maximum power which would be a setting around 4 or 5 on a current model but could be higher on an older model). Then 0.1g of water is added drop-wise across the surface of the powder. The powder is stirred for 5 minutes then stored in a sealed glass jar for 24 hours at ambient temperature, after which it is analysed by sieving.

For powders to pass the agglomeration test, no "sticky" agglomerates should be seen, and the powder should remain completely free flowing. There may be some increase in the weight % of particles in the 500-710 µm range, and a corresponding decrease in the weight % of particles below 500 µm. The weight % of particles in the 500-710 µm range should not increase by more than 30%.

The above procedure was adopted with the following products:
- Tide^{™} Free (perfume free), produced by Procter and Gamble, sold in the USA; phosphate free, and uses zeolite to soften water.
- Ariel^{™} Mild & Rein (standard or regular powder), produced by Procter and Gamble, sold in Germany (15-30% zeolite).
- Neutral^{™}, produced by Sara Lee, sold in The Netherlands (15-30% zeolite).
- Bonux^{™} powder, produced by Procter & Gamble, sold in Poland. Contains phosphate as principle water softening agent.
- Le Chat^{™} Perfect, produced by Henkel, sold in France (declared to contain carbonate, silicate and < 5% polycarboxylate, but no declaration of phosphate or zeolite on the ingredient label).

After the agglomeration test, no "sticky" agglomerates were seen, and the powder remained completely free flowing. There was some increase in the weight % of particles in the 500-710 µm range, and a corresponding decrease in the weight % of particles below 500 µm, but always less than 30%.

When significantly more water was added and the procedure above repeated, Tide, Ariel, Bonux and Le Chat were still completely satisfactory in terms of the absence of very large or "sticky" agglomerates, and being free flowing powders.

The distribution of particle size with the product Le Chat, before and after the addition of 0.52 g water to 100 g powder in the manner described above is given below.

| 100 g Le chat perfect | | | 100 g of Le chat perfect | | |
|---|---|---|---|---|---|
| | Weight (g) | % | 0.52g of water added | Weight (g) | % |
| > 710µm | 34.36 | 34.39 | > 710 µm | 32.5 | 32.6 |
| 500-710 µm | 51.47 | 51.52 | 500-710 µm | 57.6 | 57.7 |
| 355-500 µm | 9.08 | 9.09 | 355-500 µm | 8.22 | 8.2 |
| 250-355 µm | 2.95 | 2.95 | 250-355 µm | 1.49 | 1.5 |
| 100-250 µm | 2.04 | 2.04 | 100-250 µm | | |
| 50-100 µm | | | 50-100 µm | | |
| Total | 99.9 | | Total | 99.81 | |

The distribution of particle size with the product Neutral colour, before and after the addition of 0.52g water to 100g powder in the manner described above is given below.

| 100g Neutral Colour | | | 100g Neutral Colour | | |
|---|---|---|---|---|---|
| | Weight (g) | % | 0.52g of water added | Weight (g) | % |
| > 710µm | 26.00 | 26.1 | > 710µm | 25.60 | 25.7 |
| 500-710 µm | 23.60 | 23.7 | 500-710 µm | 56.04 | 56.3 |
| 355-500 µm | 20.66 | 20.8 | 355-500 µm | 16.07 | 16.2 |
| 250-355 µm | 19.94 | 20.0 | 250-355 µm | 2.08 | 2.1 |
| 100-250 µm | 9.29 | 9.3 | 100-250 µm | | |
| 50-100 µm | | | 50-100 µm | | |
| Total | 99.49 | | Total | 99.79 | |

In the case of Neutral, with 0.52g of water, the "agglomeration test" is failed.

### Example 5 - Solid Household Care Composition: Tumble dryer sheet

Tumble drier formulations of the following type were prepared:

| | TDS 1 | TDS2 | TDS3 | TDS4 |
|---|---|---|---|---|
| STEPANTEX HTS-100 from STEPAN (g) | 22 | 22 | 22 | 22 |
| Perfume composition n°2 (g) | 0.87 | | | |
| Perfume composition n°2 encapsulated (g of fragrance) I | | 0.87 | | |
| Perfume composition n°3 | | | 0.83 | |
| Perfume composition n°3 encapsulated (g of fragrance) | | | | 0.83 |

1.35 g of each of the formulations TDS1-4 were applied in spots to single clean Tumble Dryer Sheets (Bounce Free sheets 16.2 x 22.8 cm produced by Procter & Gamble and sold in USA). The resultant sheets were then individually placed between 2 clean pieces of aluminum foil, and gently melted with an iron at low heat to spread the formulations more evenly across the Tumble Dryer Sheet. Care was taken to use minimal pressure with the iron. The Sheets were left to cool, carefully separated.

Two standard 40°C European cotton cycle washes were performed with a detergent without fragrance at recommended dose (6.8 g/I), using a 2.5 Kg towel load. From each wash, half of the load was placed in a tumble drier with a sheet loaded with free fragrance (TDS1 or TDS3) and the other half was placed in another tumble drier with the sheet loaded with fragrance capsules (TDS2 or TDS4). The loads were tumble dried to "ready to iron".

| | TDS1 | TDS2 | TDS3 | TDS4 |
|---|---|---|---|---|
| Amount of perfume composition n° 2 on dry towel (µg) | 96 | 850 | | |
| Amount of perfume composition n° 3 on dry towel (µg) | | | 1600 | 2600 |

The above example shows that not only many capsules are not damaged during the tumble drying process but also a lot more fragrance are deposited on cloth when the fragrance is encapsulated. This deposition enhancement is olfactively perceivable when the towels are rubbed together and smelt.

### Example 6 : A solid Personal Care composition: Deo-stick

A dispersion containing 40% of core shell capsule with perfume composition n°2 was mixed at 1% by weight in a Deo stick base according to the formulation below. Samples were left for 24 h at room temperature.

| Ingredients | % W/W | INCI designation | Supplier |
|---|---|---|---|
| DOW CORNING 245 | 53.00 | CYCLOMETHICONE | DOW CORNING |
| LANETTE 22 | 23.00 | BEHENYL ALCOHOL | COGNIS |
| ISOPROPYLE MYRISTATE | 3.00 | ISOPROPYL MYRISTATE | LAMBERT RIVIERE |
| REACH 501 | 20.00 | ALUMINUIM CHLORHYDRATE | REHEIS |
| Dispersion CD105 | 1.00 | Perfume composition n° 2 | |

The capsules were homogeneously distributed in the base. They also did not initiate any property modification of the base.

### Example 7 : A liquid Personal Care composition: Hair gel

A dispersion containing 40% of core shell capsule with Perfume composition n°2 (Table 4) - CD105 - was mixed at 0.5% by weight in a hair gel base (see formula below). Samples were left for 24 h at room temperature.

### Hair gel base

| Ingredients | % W/W | INCI designation | Supplier |
|---|---|---|---|
| AQUA | 86.85 | AQUA | |
| KATHON CG | 0.05 | METHYLCHLOROISOTHIA ZOLINONE & METHYLISOTHIAZOLINON E | SEPPIC |
| EDETA B | 0.10 | EDTA | BASF |
| CARBOPOL ULTREZ 10 | 1.00 | CARBOMER | GOODRICH |

| Ingredients | %W/W | INCI designation | Supplier |
|---|---|---|---|
| ETHANOL 96 | 10.00 | ETHANOL | |
| LUVISKOL VA 64 powder | 1.50 | PVP / VA COPOLYMER | BASF |
| Dispersion CD 105 | 0.5 | Perfume composition n° 2 | |

The capsules were homogeneously distributed in the base. They also did not initiate any property modification of the base.

### Example 8 : Liquid household cleaning and care compositions: Liquid detergent and liquid fabric conditioner.

The following products were prepared, and all had satisfactory physical appearance after preparation, and on storage.

### Example 8.1:

The core shell capsule dispersion CD105 was mixed at 1.4% by weight in a Lenor Premium Rinse conditioner made by Procter & Gamble and sold in Germany. The sample was agitated for 1h on a roller-bed and then left for 24h at room temperature.

### Example 8.2:

The core shell capsule dispersion of perfume composition n°2 was mixed at 1.4% by weight in Vernel Rinse conditioner, made by Henkel and sold in Spain. The sample was agitated for 1h on a roller-bed and then left for 24 h at room temperature.

### Example 8.3:

The core shell capsule dispersion of perfume composition n°2 was mixed at 1.4% by weight in Dash Alpina liquid washing machine detergent made by Protect & Gamble and sold in Italy. The sample was agitated for 1h on a roller-bed and then left for 24 h at room temperature.

### Example 9

This example shows a fragrance composition of the invention which may form the core of a capsule. It is a floral tea accord suited to fabric cleaning or conditioning products

| Ingredient | CAS NO | ClogP | Weight % |
|---|---|---|---|
| Alpha pinene | 80-56-8 | 4.70 | 0.50 |
| Eucalyptol | 470-82-6 | 2.83 | 4.50 |
| Dihydromyrcenol | 18479-58-8 | 3.03 | 45.00 |
| Linalool | 78-70-6 | 2.75 | 30.00 |
| Benzyl Acetate | 140-11-4 | 1.96 | 1.00 |
| Ethyl Benzoate | 93-89-0 | 2.64 | 0.70 |
| C-10 alcohol | 112-30-1 | 4.0 | 0.30 |
| Dimethylbenzylcarbinyl acetate | 151-05-3 | 2.99 | 14.00 |
| Phenylethyl-2-methylbutyrate | 24817-51-4 | 3.74 | 2.00 |
| Hexyl benzoate | 6789-88-4 | 4.76 | 0.50 |
| Acetyl iso Eugenol | 93-29-8 | 2.48 | 1.50 |

### Example 10

This example illustrates a capsule core composition comprising 75% of a fragrance having the formulation in the table and 25% of a benefit agent cooling agent 10™. The fragrance note in this case is a fresh spicy note

| Ingredient | CAS NO | ClogP | Weight % |
|---|---|---|---|
| Linalool | 78-70-6 | 2.75 | 50.00 |
| Benzyl acetate | 140-11-4 | 1.96 | 1.875 |
| Verdox | 88-41-5 | 4.06 | 1.00 |
| Damascenone | 23696-85-7 | 4.27 | 0.25 |
| Cyclacet | 5413-60-5 | 2.88 | 10.00 |
| Beta ionone | 14901-07-6 | 3.71 | 4.375 |
| Iso super E | 54464-57-2 | 4.85 | 1.25 |
| Musk T | 105-95-3 | 3.02 | 31.25 |

### Example 11

This example illustrates a capsule core composition with a more floral carnation accord suited to a range of household and personal care products.

| Ingredient | CAS NO | ClogP | Weight % |
|---|---|---|---|
| Acetophenone | 98-86-2 | 1.58 | 0.50 |
| Methyl salicylate | 119-36-8 | 2.33 | 0.10 |
| Koavone | 81786-73-4 | 3.48 | 20.00 |
| Phenylacetaldehyde dimethyl acetal | 101-48-4 | 1.57 | 2.50 |
| Eugenol | 97-53-0 | 2.40 | 0.90 |
| Hedione | 24851-98-7 | 2.91 | 75.00 |
| Orbitone | 37609-25-9 | 5.97 | 1.00 |

### Example 12

This example illustrates a capsule core composition which is a floral accord suited to a range of household and personal care products.

| Ingredient | CAS NO | ClogP | Weight % |
|---|---|---|---|
| Fruitate | 80657-64-3/80623-07-0 | 3.37 | 2.00 |
| Styrallyl acetate | 93-92-5 | 2.28 | 3.00 |
| Benzyl acetate | 140-11-4 | 1.96 | 5.00 |
| Beta ionone | 14901-07-6 | 3.71 | 5.00 |
| Dimethylbenzylcarbinyl acetate | 151-05-3 | 2.99 | 5.00 |
| Hedione | 24851-98-7 | 2.91 | 15.00 |
| Linalool | 78-70-6 | 2.75 | 15.00 |
| Dihydromyrcenol | 18479-58-8 | 3.03 | 20.0 |
| Musk T | 105-95-3 | 3.02 | 30.00 |

### Example 13

This is a green floral accord suitable for household or personal care products. It is also free from any of the 26 fragrance ingredients designated as allergens in the directives and regulations governing cosmetic and detergent products for sale within the European Union.

| Ingredient | CAS NO | ClogP | Weight % |
|---|---|---|---|
| Undecavertol | 81782-77-6 | 3.89 | 5.00 |
| Dec-9-en-1-ol | 13019-22-2 | 3.51 | 5.00 |
| Koavone | 81786-73-4 | 3.48 | 5.00 |
| Dimethylbenzylcarbinyl acetate | 151-05-3 | 2.99 | 10.00 |
| Oxalide T | 1725-01-5 | 3.50 | 10.00 |
| Cyclacet | 5413-60-5 | 2.88 | 15.00 |
| Dihydromyrcenol | 18479-58-8 | 3.03 | 15.00 |
| Hedione | 24851-98-7 | 2.91 | 15.00 |
| Musk T | 105-95-3 | 3.02 | 20.00 |

## Claims

1. A core shell capsule containing in the core an oil or waxy solid, wherein the oil or waxy solid comprises by weight:
• 80-100% of a perfume composition, which is a mixture of at least two perfume ingredients wherein:
a) aldehydes, including alpha beta unsaturated aldehydes constitute 0-20% by weight of the perfume composition;
b) primary or secondary amines constitute 0-10% by weight of the perfume composition;
c) 0-25% by weight of the perfume composition has ClogP >4.0;
d) 0-20% by weight of the perfume composition has ClogP >5.0; and
e) 0-20% by weight of the perfume composition has ClogP <2.0;
• 0 - 20% by weight of benefit agents other than perfume ingredients;
where the capsule is an aminoplast capsule; and
where ClogP is calculated using the CLOGP program available within the Chemoffice Ultra Software version 9.

2. A core shell capsule according to Claim 1, where the shell is constituted of 50-100% formaldehyde-melamine or formaldehyde-melamine-urea or formaldehyde-urea condensation polymer, or of the corresponding partially etherified formaldehyde condensation polymers, preferably as methyl ethers.

3. A core shell capsule according to claim 1 wherein the oil or waxy solid contains 0-1% of perfume ingredients which are selected among:
i. the aldehydes selected among the group consisting of amyl cinnamic aldehyde; citral (CAS 005392-40-5); hydroxy-citronellal; cinnamic aldehyde; hydroxymethylpentyl-cyclohexenecarboxaldehyde; 2-(4-tert-butylbenzyl) propionaldehyde; hexyl cinnamic aldehyde; phenyl acetaldehyde; trans-2-heptenal; 2,4-dihydroxy-3-methyl benzaldehyde; Benzaldehyde; Crotonaldehyde E (CAS 123-73-9); and furfural (CAS 98-01-1);
ii. the perfume ingredients having a ClogP > 4 selected among the group consisting of Benzyl salicylate, Benzyl cinnamate, Farnesol (CAS 4602-84-0), d-Limonene, l-Limonene, D, L-Limonene (racemic), 3-Methyl-4-(2,6,6-trimethyl-2-cyclohexen-1-yl)-3-buten-2-one, Cyclowood (CAS 13019-04-0), Polysantol (CAS 107898-54-4),
iii. the perfume ingredients having a ClogP < 2 selected among the group consisting of benzyl alcohol, Cinnamyl alcohol, Coumarin; Anisyl alcohol; Acetal E71 (CAS 105-57-7), acetophenone; Sec-Butyl acetate; tert-Butyl acetate; n-Butyl acetate; isoButyl acetate, p-Cresol; Ethyl acetate; Ethyl propionate; Propyl acetate; Ethyl propionate; Propyl acetate; Benzyl cyanide.

4. A core shell capsule containing an oil or waxy solid according to Claim 1 where the oil or waxy solid does not contain any of the following: phthalate esters, nitro musks, polycyclic musks, cashmeran, geranyl nitrile, safrole, estragol, methyl eugenol, halogen containing perfumery materials, and carbitol ethers defined as R-(OCH₂CH₂)ₙ-OR¹ where n= 1,2 or 3, R = C₁-C₇ alkyl or phenyl or alkyl substituted phenyl and R¹ is H or C₁-C₇ alkyl with the exception of carbitols based on propyl groups.

5. A core shell capsule containing an oil or waxy solid according to Claim 1 where the oil or waxy solid has a peroxide value of 0-20 millimoles peroxide / litre, preferably 0-10 millimoles peroxide / litre, and even more preferably 0-1 millimoles peroxide / litre.

6. A core shell capsule containing an oil or waxy solid according to Claim 1 where the core shell capsule has a particle size of 1 µm to 100 µm and preferably 5-70 µm with a shell wall thickness of between 0.025 and 1.0 µm.

7. Use of a core shell capsule of Claims 1 to 2 for the manufacture of a personal care composition which may be in the form of a liquid, gel or solid containing a surfactant, said personal care composition being most preferably bars, powders or solid sticks.

8. Use of a capsule of Claims 1 to 2 for the manufacture of a solid household cleaning or care composition containing a surfactant, said composition being preferably a powder, bar, dry aerosol, tablet or coated non-woven substrate.

9. A liquid household cleaning or care conditioning composition containing a surfactant and a capsule of Claims 1 to 2 which may be in the form of a liquid, gel, capsule, wet aerosol or coated non-woven substrate.

10. A powder composition produced by spray drying capsules according to Claims 1 to 2 in a mixture with inorganic salts, and optionally a binder and/or surfactant.

11. A method of delivering perfume to surfaces which comprises contacting the surfaces with compositions, optionally diluted with water, of any of claims 9 to 10.

## Patentansprüche

1. Kern-Hüllen-Kapsel, welche in dem Kern ein Öl oder einen wachsartigen Feststoff enthält, wobei das Öl oder der wachsartige Feststoff gewichtsmäßig umfaßt:
• 80 - 100 % einer Parfümzusammensetzung, welche ein Gemisch von wenigstens zwei Parfüminhaltsstoffen ist, wobei:
a) Aldehyde, einschließlich alpha-beta-ungesättigten Aldehyden 0 - 20 Gew.-% der Parfümzusammensetzung bilden;
b) primäre oder sekundäre Amine 0 - 10 Gew.-% der Parfümzusammensetzung bilden;
c) 0 - 25 Gew.-% der Parfümzusammensetzung einen ClogP > 4.0 haben
d) 0 - 20 Gew.-% der Parfümzusammensetzung einen ClogP > 5.0 haben
e) 0 - 20 Gew.-% der Parfümzusammensetzung einen ClogP < 2.0 haben
• 0 - 20 Gew.-% zuträgliche Mittel, die von Parfüminhaltsstoffen verschieden sind;
wobei die Kapsel eine Aminoplastkapsel ist; und
wobei CLOGP wird durch das "CLOGP" -Programm berechnet, das in der Chemoffice Ultra Software Version 9 verfügbar ist.

2. Kern-Hüllen-Kapsel nach Anspruch 1, wobei die Hülle gebildet ist aus 50 - 100 % Formaldehyd-Melamin- oder Formaldehyd-Melamin-Harnstoff- oder Formaldehyd-Harnstoff-Kondensationspolymer oder den entsprechenden vorzugsweise als Methylether teiletherifizierten Formaldehyd-Kondensationspolymeren.

3. Kern-Hüllen-Kapsel nach Anspruch 1, wobei das Öl oder der wachsartige Feststoff 0 - 1 % Parfüminhaltsstoffe enthält, welche ausgewählt sind unter:
i. den Aldehyden, die gewählt sind aus der Gruppe, die besteht aus Amylzimtaldehyd, Citral (CAS 005392-40-5), Hydroxy-Citronellal, Zimtaldehyd, Hydroxymethylpentyl-Cyclohexencarboxaldehyd, 2-(4-tert.-Butylbenzyl)-Propionaldehyd, Hexylzimtaldehyd, Phenylacetaldehyd, trans-2-Heptenal, 2,4-Dihydroxy-3-Methyl-Benzaldehyd, Benzaldehyd, Crotonaldehyd E (CAS 123-73-9) und Furfural (CAS 98-01-1);
ii. Parfüminhaltsstoffen mit einem ClogP > 4, gewählt aus der Gruppe, die besteht aus Benzylsalicylat, Benzylcinnamat, Farnesol (CAS 4602-84-0), d-Limonen, l-Limonen, D-, L-Limonen (racemisch), 3-Methyl-4(2,6,6-Trimethyl-2-Cyclohexen-1-yl)-3-Buten-2-on, Cyclowood (CAS 13019-04-0), Polysantol (CAS 107898-54-4);
iii. Parfüminhaltsstoffen mit einem ClogP < 2, gewählt aus der Gruppe, die besteht aus Benzylalkohol, Cinnamylalkohol, Coumarin, Anisylalkohol, Acetal E71 (CAS 105-57-7), Acetophenon, sec.-Butylacetat, tert.-Butylacetat, n-Butylacetat, Iso-Butylacetat, p-Cresol, Ethylacetat, Ethylpropionat, Propylacetat, Ethylpropionat, Propylacetat, Benzylcyanid.

4. Kern-Hüllen-Kapsel, die ein Öl oder einen wachsartigen Feststoff enthält, nach Anspruch 1, wobei das Öl oder der wachsartige Feststoff nicht Folgendes enthält: Phthalat-Ester, Nitromoschus, polyzyklischen Moschus, Kaschmir, Geranylnitril, Safrol, Estragol, Methyleugenol, halogenhaltige Parfümmaterialien und Carbitolester, die definiert sind als R-(OCH₂CH₂)ₙ-OR¹, wobei n = 1, 2 oder 3, R = C₁-C₇-Alkyl oder Phenyl oder alkylsubstituiertes Phenyl ist und R¹ H oder C₁-C₇-Alkyl ist, mit der Ausnahme von Carbitolen, die auf Propylgruppen basieren.

5. Kern-Hüllen-Kapsel, welche ein Öl oder einen wachsartigen Feststoff enthält, nach Anspruch 1, wobei das Öl oder der wachsartige Feststoff einen Peroxidwert von 0 - 20 mmol Peroxid/Liter, vorzugsweise 0 - 10 mmol Peroxid/Liter und noch mehr bevorzugt 0 - 1 mmol Peroxid/Liter hat.

6. Kern-Hüllen-Kapsel, welche ein Öl oder einen wachsartigen Feststoff enthält, nach Anspruch 1, wobei die Kern-Hüllen-Kapsel eine Partikelgröße von 1 µm bis 100 µm und bevorzugt 5 - 70 µm mit einer Hüllwanddicke zwischen 0,025 und 1,0 µm aufweist.

7. Verwendung einer Kern-Hüllen-Kapsel nach Anspruch 1 bis 2 zur Herstellung einer Körperpflegezusammensetzung, welche in Form einer Flüssigkeit, eines Gels oder eines Feststoffs vorliegen kann, enthaltend ein Tensid, wobei die Körperpflegezusammensetzung am bevorzugtesten Stäbchen, Pulver oder Feststoffstäbchen sind.

8. Verwendung einer Kapsel nach Anspruch 1 bis 2 zur Herstellung einer festen Haushaltsreinigungs- oder Pflegezusammensetzung, welche ein Tensid enthält, wobei die Zusammensetzung bevorzugt ein Pulver, ein Stab, ein Trockenaerosol, eine Tablette oder ein beschichtetes Vliessubstrat ist.

9. Flüssige Haushaltsreinigungs- oder Pflegeaufbereitungszusammensetzung, die ein Tensid und eine Kapsel nach Anspruch 1 bis 2 enthält, welche in Form einer Flüssigkeit, eines Gels, einer Kapsel, eines Nassaerosols oder eines beschichteten Vliessubstrats vorliegen kann.

10. Pulverzusammensetzung, die erzeugt ist durch Sprühtrocknung von Kapseln nach Anspruch 1 bis 2 in einem Gemisch mit anorganischen Salzen und gegebenenfalls einem Bindemittel und/oder Tensid.

11. Verfahren zur Parfümabgabe an Oberflächen, welches das Kontaktieren der Oberflächen mit Zusammensetzungen, gegebenenfalls verdünnt mit Wasser, von einem der Ansprüche 9 bis 10 umfaßt.

## Revendications

1. Capsule noyau-enveloppe contenant dans le noyau un solide huileux ou cireux, dans laquelle le solide huileux ou cireux comprend en poids :
• 80-100 % d'une composition de parfum, laquelle est un mélange d'au moins deux ingrédients de parfum dans lequel :
a) les aldéhydes, comprenant des aldéhydes alpha-bêta-insaturés, constituent 0-20 % en poids de la composition de parfum ;
b) les amines primaires ou secondaires constituent 0-10 % en poids de la composition de parfum ;
c) 0-25 % en poids de la composition de parfum présente un ClogP > 4,0 ;
d) 0-20 % en poids de la composition de parfum présente un ClogP > 5,0 ;
e) 0-20 % en poids de la composition de parfum présente un ClogP < 2,0 ;
• 0-20 % en poids d'agents bénéfiques différents des ingrédients de parfum ;
la capsule étant une capsule aminoplaste ; et
ClogP est calculé en utilisant le programme CLOGP disponible dans le logiciel Chemoffice Ultra version 9.

2. Capsule noyau-enveloppe selon la revendication 1, dans laquelle l'enveloppe est constituée de 50-100 % de polymère de condensation de formaldéhyde-mélamine ou de formaldéhyde-mélamine-urée ou de formaldéhyde-urée, ou de polymères de condensation de formaldéhyde partiellement éthérifiés correspondants, de préférence sous la forme de méthyléthers.

3. Capsule noyau-enveloppe selon la revendication 1, dans laquelle le solide huileux ou cireux contient 0-1 % d'ingrédients de parfum qui sont choisis parmi :
i. les aldéhydes choisis dans le groupe constitué de l'aldéhyde amylcinnamique ; du citral (CAS 005392-40-5) ; de l'hydroxy-citronellal ; de l'aldéhyde cinnamique ; de l'hydroxyméthylpentyl-cyclohexènecarboxaldéhyde ; du 2-(4-tert-butylbenzyl)propionaldéhyde ; de l'aldéhyde hexylcinnamique ; du phénylacétaldéhyde ; du trans-2-hepténal ; du 2,4-dihydroxy-3-méthylbenzaldéhyde ; du benzaldéhyde ; du crotonaldéhyde E (CAS 123-73-9) ; et de furfural (CAS 98-01-1) ;
ii. les ingrédients de parfum présentant un ClogP > 4 choisis dans le groupe constitué du salicylate de benzyle, du cinnamate de benzyle, du Farnésol (CAS 4602-84-0), du D-limonène, du L-limonène, du D, L-limonène (racémique), du 3-méthyl-4-(2,6,6-triméthyl-2-cyclohexèn-1-yl)-3-butèn-2-one, du Cyclowood (CAS 13019-04-0), du Polysantol (CAS 107898-54-4),
iii. les ingrédients de parfum présentant un ClogP < 2 choisis dans le groupe constitué de l'alcool benzylique ; de l'alcool cinnamylique ; de la coumarine ; de l'alcool anisylique ; de l'Acétal E71 (CAS 105-57-7) ; de l'acétophénone ; de l'acétate de sec-butyle ; de l'acétate de tert-butyle ; de l'acétate de n-butyle ; de l'acétate d'iso-butyle ; du p-crésol ; de l'acétate d'éthyle ; du propanoate d'éthyle ; de l'acétate de propyle ; du propanoate d'éthyle ; de l'acétate de propyle ; du cyanure de benzyle.

4. Capsule noyau-enveloppe contenant un solide huileux ou cireux selon la revendication 1, dans laquelle le solide huileux ou cireux ne contient aucun des composés suivants : esters de phtalate, muscs nitro, muscs polycycliques, cashmeran, nitrile de géranyle, safrole, estragol, méthyleugénol, matières de parfumerie contenant des halogènes, et carbitoléthers définis par R-(OCH₂CH₂)ₙ-OR¹ dans laquelle n = 1, 2 ou 3, R = (C₁-C₇)alkyle ou phényle ou phényle substitué par un alkyle et R¹ est H ou un (C₁-C₇)alkyle à l'exception des carbitols à base de groupe propyle.

5. Capsule noyau-enveloppe contenant un solide huileux ou cireux selon la revendication 1, dans laquelle le solide huileux ou cireux présente une valeur peroxyde de 0-20 mmol de peroxyde/l, de préférence de 0-10 mmol de peroxyde/l et de préférence encore de 0-1 mmol de peroxyde/l.

6. Capsule noyau-enveloppe contenant un solide huileux ou cireux selon la revendication 1, dans laquelle la capsule noyau-enveloppe présente une taille de particules de 1 µm à 100 µm et de préférence de 5-70 µm avec une épaisseur de paroi d'enveloppe de 0,025 à 1,0 µm.

7. Utilisation d'une capsule noyau-enveloppe selon la revendication 1 ou 2 pour la fabrication d'une composition de soin corporel qui peut être sous la forme d'un liquide, d'un gel ou d'un solide contenant un tensioactif, ladite composition de soin corporel étant de préférence sous forme de barres, de poudres ou de sticks solides.

8. Utilisation d'une capsule selon la revendication 1 ou 2 pour la fabrication d'une composition solide pour le nettoyage ménager ou le soin contenant un tensioactif, ladite composition étant de préférence une poudre, une barre, un aérosol sec, une tablette ou un substrat non tissé enrobé.

9. Composition liquide de nettoyage ménager ou de soin contenant un tensioactif et une capsule selon la revendication 1 ou 2 qui peut être sous la forme d'un liquide, d'un gel, d'une capsule, d'un aérosol humide ou d'un substrat non tissé enrobé.

10. Composition de poudre produite par séchage par pulvérisation de capsules selon la revendication 1 ou 2 en mélange avec des sels inorganiques et éventuellement un liant et/ou un tensioactif.

11. Procédé pour délivrer un parfum sur des surfaces qui comprend la mise en contact des surfaces avec des compositions, éventuellement diluées avec de l'eau, selon l'une des revendications 9 et 10.
